# EUROPÄISCHE PATENTSCHRIFT

(11) **EP 0 643 133 B1**
(45) Veröffentlichungstag und Bekanntmachung des Hinweises auf die Patenterteilung: **21.11.2001**
(21) Anmeldenummer: 94113110.4
(22) Anmeldetag: 23.08.1994
(51) Int. Cl.: C12N 15/55, C12N 9/86, C12N 1/21, C12N 1/19, C12P 21/02, C12P 13/02, C12P 41/00

(54) **Rekombinante D-Hydantoinase, Verfahren zur Herstellung und Verwendung**
Recombinant D-hydantoinase, process for its preparation and its use
D-hydantoinase récombinante, procédé de sa préparation et son utilisation

(30) Priorität: 27.08.1993 DE 4328829
(43) Veröffentlichungstag der Anmeldung: 15.03.1995
(73) Patentinhaber: Roche Diagnostics GmbH, 68298 Mannheim (DE)
(72) Erfinder: Burtscher, Dr. Helmut, D-82392 Habach (DE); Lang, Gunter, D-82327 Tutzing (DE); Popp, Dr. Friedrich, D-82404 Sindelsdorf (DE)

(56) Entgegenhaltungen:
- EP-A- 0 046 186
- EP-A- 0 152 977
- EP-A- 0 219 034
- EP-A- 0 515 698
- JP-A- 4 325 093

## Beschreibung

Gegenstand der Erfindung ist eine neue rekombinante D-Hydantoinase, ein Verfahren zu ihrer Herstellung und ihre Verwendung.

D-Hydantoinasen (Dihydropyrimidinasen, EC.3.5.2.2) werden zur Herstellung von D-N-Carbamoyl-alpha-aminosäuren verwendet. Diese Verbindungen sind wichtige Zwischenprodukte zur Herstellung von D-Aminosäuren (Morin et al., Appl. Microbiol. 35, 536 - 540 (1991), EP-B 0 219 034). D-Aminosäuren wiederum sind wertvolle Ausgangsstoffe für die Synthese der Seitenketten von Penicillinen und halbsynthetischen Cephalosporinen. Die Herstellung von D-N-Carbamoyl-alpha-aminosäuren erfolgt vorteilhaft bei höheren Temperaturen, da dann die Hydantoine besser löslich sind, die Racemisierung schneller erfolgt und auch die Umsetzung beschleunigt wird. Aus diesem Grund besteht ein Bedarf an thermostabilen D-Hydantoinasen.

Eine D-Hydantoinase, welche bei hohen Temperaturen (40 - 90°C) aktiv ist, kann aus thermophilen Mikroorganismen erhalten werden (DE-A 30 31 151). Diese thermophilen Mikroorganismen sind jedoch schwer kultivierbar und wachsen schlecht. Außerdem wird die D-Hydantoinase von diesen Mikroorganismen nur in sehr geringen Mengen produziert.

In der EP-B 0 219 034 ist eine rekombinante D-Hydantoinase beschrieben. Bei der Expression der in der EP-B 0 219 034 beschriebenen DNA-Sequenzen wird jedoch D-Hydantoinaseaktivität nur in geringer Menge erhalten.

Aufgabe der vorliegenden Erfindung war es, eine rekombinante D-Hydantoinase mit weiter verbesserter Temperaturstabilität in großen Mengen zur Verfügung zu stellen.

Diese Aufgabe wird gelöst durch eine DNA-Sequenz, welche für ein Protein codiert, welches D-Hydantoinasenaktivität besitzt und gekennzeichnet ist durch die Aminosäuresequenz SEQ ID NO 1.

Das Protein
a) besitzt ein pH-Optimum bei ca. 8,2, ein Optimum der pH-Stabilität zwischen ca. 6,5 und 9,0,
b) weist in 50 mmol/l Trispuffer, pH 7,8, bei einer Konzentration von 15 mg/ml nach 20 Minuten bei 60°C noch ca. 100 % der Ausgangsaktivität und nach 20 Minuten bei 65°C noch ca. 80 % der Ausgangsaktivität auf,
c) ist ein nicht natürlich vorkommendes Polypeptid,
d) ist das Produkt einer prokaryontischen Expression einer exogenen DNA.

Es hat sich überraschenderweise gezeigt, daß das erfindungsgemäße Enzym rekombinant in Prokaryonten in großen Mengen herstellbar ist, gut löslich ist und eine hohe Aktivität und gute Temperaturstabilität besitzt.

Die erfindungsgemäße rekombinante D-Hydantoinase unterscheidet sich von dem nativen Enzym (Wildtyp-Enzym) aus beispielsweise thermophilem Bacillus (DE-OS 30 31 151), von dem in JP-A 4/325 093 und von dem in der EP-B 0 219 034 beschriebenen Enzym in der Aminosäuresequenz am C-Terminus.

Das erfindungsgemäße Enzym ist 12 Aminosäuren kürzer als das Wildtyp-Enzym und unterscheidet sich in der Sequenz der letzten 6 Aminosäuren. Von dem in JP-A 4/325 093 beschriebenen Enzym unterscheidet sich das erfindungsgemäße Enzym in 33 Aminosäuren; außerdem ist letzteres 11 Aminosäuren kürzer. Gegenüber dem in der EP-B 0 219 034 beschriebenen Enzym ist das erfindungsgemäße Enzym 8 Aminosäuren länger und unterscheidet sich in der Sequenz der letzten 30 Aminosäuren.

Als erfindungsgemäße D-Hydantoinasen sind auch solche Proteine zu verstehen, welche sich in ihrer Aminosäuresequenz geringfügig von SEQ ID NO 1 unterscheiden. Dabei können Aminosäuren ausgetauscht, deletiert, derivatisiert oder addiert werden.

Zur rekombinanten Herstellung der erfindungsgemäßen D-Hydantoinase wird eine DNA verwendet, welche für ein Protein mit D-Hydantoinasenaktivität codiert und ausgewählt ist aus der Gruppe
a) der in Sequenz ID NO 2 gezeigten DNA-Sequenz oder der dazu komplementären DNA-Sequenz,
b) DNA-Sequenzen, die aufgrund der Degeneration des genetischen Codes für ein Protein codieren, welches auch von einer der in a) definierten Sequenzen codiert wird.

Vorzugsweise wird eine DNA-Sequenz der Sequenz ID NO 2 verwendet.

Die DNA-Sequenzen können in einer dem Fachmann geläufigen Weise geringfügig modifiziert werden. Beispielsweise können degenerierte Codons durch andere Codons, welche für die gleiche Aminosäure codieren, ersetzt werden. Außerdem können zusätzliche Codons am 5'- und am 3'-Ende oder auch innerhalb der Sequenzen eingefügt werden oder Deletionen einzelner Codons oder Codongruppen erfolgen, solange sich die so erhaltenen DNA-Varianten von den erfindungsgemäßen Sequenzen nur geringfügig unterscheiden, mit diesen Sequenzen unter üblichen Bedingungen (Sambrook et al., Molecular Cloning, A Laboratory Manual, Cold Spring Harbor Laboratory Press, New York 1989) hybridisieren und das codierte Protein D-Hydantoinasen-Aktivität besitzt.

Ein weiterer Gegenstand der Erfindung ist ein Verfahren zur Herstellung einer rekombinanten D-Hydantoinase durch Transformation einer geeigneten prokaryontischen Wirtszelle (z. B. E.coli, Saccharomyces cerevisiae) mit einer erfindungsgemäßen DNA, welche in einem geeigneten Expressionssystem vorliegt, Kultivierung der transformierten Wirtszellen und Isolierung der gebildeten D-Hydantoinase aus den Zellen oder dem Zellüberstand.

Die Transformation der für die rekombinante Herstellung verwendeten Wirtszellen erfolgt nach bekannten Verfahren (Sambrook et al. 1989) Die transformierten Wirtszellen werden unter Bedingungen kultiviert, die eine Expression des D-Hydantoinasegens erlauben. Je nach verwendetem Expressionsvektor ist hierfür in bekannter Weise ggf. die Zugabe eines Induktors (z. B. Lactose oder Isopropyl-β-D-Thiogalactopyranosid (IPTG)) zum Kulturmedium erforderlich. Die Isolierung der rekombinanten D-Hydantoinase aus dem Zellüberstand oder den Zellen erfolgt in bekannter Weise.

Mit diesem Verfahren ist es möglich, rekombinante, aktive D-Hydantoinase in einer Ausbeute von bis zu 10⁶ U/1,5 kg Biomasse zu erhalten.

Aufschluß und Aufreinigung der rekombinant hergestellten D-Hydantoinase können nach den dem Fachmann geläufigen Methoden durchgeführt werden. Vorzugsweise wird die nach Fermentation erhaltene Biomasse in einem Hochdruckhomogenisator aufgeschlossen, der rohe Extrakt mit Ammonsulfat und einer Inkubation bei ca. 60°C fraktioniert (Hitzeschritt).

Ein weiterer Gegenstand der Erfindung ist ein Verfahren zur Spaltung eines racemischen Hydantoins in die entsprechenden D-N-Carbamoyl-alpha-aminosäure, welches dadurch gekennzeichnet ist, daß das racemische Hydantoin mit einer erfindungsgemäßen D-Hydantoinase bei einer Temperatur von 50 - 80°C inkubiert wird und die entstandene D-N-Carbamoyl-alpha-aminosäure anschließend nach dem Fachmann geläufigen Methoden aus dem Reaktionsgemisch isoliert und ggf. gereinigt wird.

Sämtliche gentechnologische Verfahren wie z.B. Expression, DNA-Modifikation, Klonierung und Gewinnung des rekombinanten Proteins können nach den dem Fachmann geläufigen Methoden wie sie z.B. in Ausubel, F.M., et al., Current Protocols in Molecular Biology, Wiley, New York 1992; Sambrook et al. (1989) oder Davis, L.G., Methods in Molecular Biology, Elsevier, Amsterdam, NL, 1986 beschrieben sind durchgeführt werden.

Die nachfolgenden Beispiele, das Sequenzprotokoll und die Abbildung erläutern die Erfindung weiter:

### Beispiel 1

### Plasmidherstellung

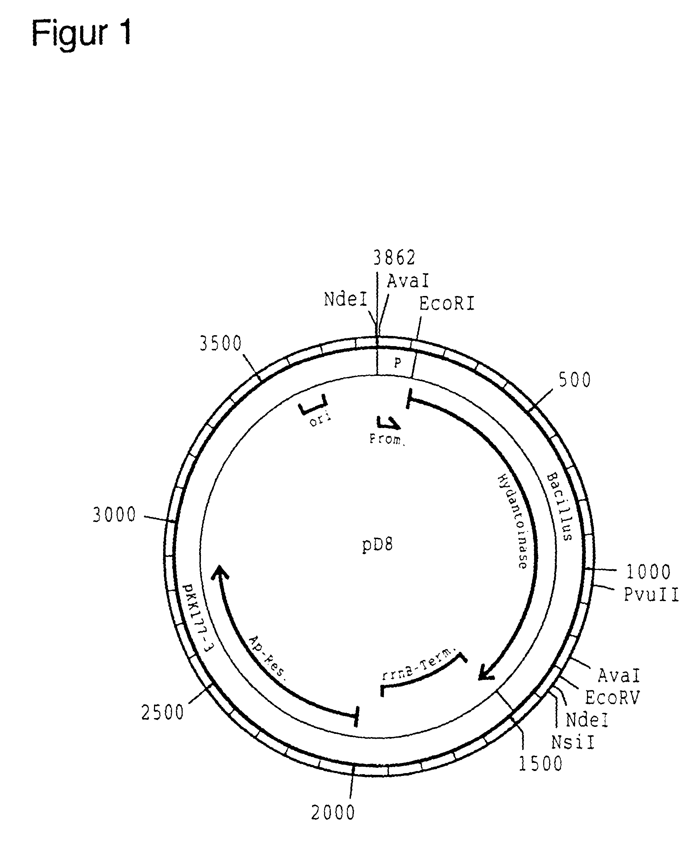

Mit Hilfe zweier Primer (Hydl und Hyd2) wird ein D-Hydantoinase-Gen aus Bacillus thermoglucodasius (Wildtyp) isoliert und nach Restriktion mit den Restriktionsenzymen EcoRI und HindIII in einen für die Expression in E. coli geeigneten Expressionsvektor (pKK177-3, DSM 3062) insertiert. Deletiert man eine Base aus der HindIII-Schnittstelle (AAGCTT -> AAGCT), so entsteht ein Leserahmen, der in ein Protein mit SEQ ID 1 übersetzt werden kann (Fig. 1).

Das resultierende Plasmid pD8 enthält SEQ ID NO 2 als proteinkodierende Sequenz unter der Kontrolle eines IPTG-induzierbaren Promotors. In diesem Plasmid kann der Promotor durch andere Promotoren wie z.B. durch den lac-Promotor, mgl-Promotor (EP-A 0 316 370) oder durch die in EP-A 0 186 069 und EP-A 0 303 925 beschriebenen Promotoren ersetzt werden.

### Beispiel 2

### Beschreibung des Fermentationsverfahrens für rekombinante Hydantoinase aus E. coli

Als Wirtsorganismus wird E.coli HB 101 (DSM 1607) verwendet, der pD8 sowie auf einem kompatiblen Plasmid das lacI-Gen enthält.

Die Anzucht der Vorkulturen erfolgte ausgehend von in Flüssig-Stickstoff gelagerten Pailletten in LB Medium mit doppeltem Selektionsdruck (Kanamycin und Ampicillin).

Das Animpfvolumen für die Hauptkultur beträgt 1 - 10 Vol %. Hauptbestandteile des HK-Mediums sind Hefeextrakt und Glucose. Das Medium wird vor dem Beimpfen mit K₂HPO₄ auf pH 7,6 - 7,8 eingestellt.

Weitere essentielle Medienbestandteile sind Mn- und Mg-Salze. Sie sind notwendig für die Aktivität und Stabilität der Hydantoinase. Die Salze werden getrennt sterilisiert und separat zum Medium zugegeben. Ein nur geringer Anteil (ca. 20 %) wird vorgelegt, der Hauptanteil wird über eine Glucosedosage, die als Säureregulans eingesetzt wird, zugegeben.

Zur Vermeidung der Bildung von Inclusion Bodies (IBs) wird erst bei einer OD₅₇₈ = 10 mit wenig IPTG (<1 mmol) induziert. Die Fermentationstemperatur beträgt 32°C. Zusätzlich erfolgt ab einer OD₅₇₈ = 30 eine limitierende Dosage von Hefeextrakt zur Begrenzung der Wachstumsgeschwindigkeit.

Durch die Regulierung und Limitierung der spez. Wachstumsrate über die Dosagegeschwindigkeit läßt sich die IB-Bildung nahezu vollständig unterdrücken, und trotzdem kann ein hoher Biomasseertrag erzielt werden. Der pH Wert wird bei 7,0 - 7,2 reguliert. Um unerwünschte Säurebildung zu unterdrücken, wird über die Rührerdrehzahl die Zuluftregulierung, die Dosagegeschwindigkeit und/oder Druck der gelöst-Sauerstoffwert pO₂ > 10 % gehalten.

Nach ca. 40 Std. Fermentationszeit wird eine OD₅₇₈ von 120 - 140 erreicht. Dies bedeutet eine Biomasseausbeute von 45 - 50 g TG/l. Die Hydantoinaseaktivität liegt dabei > 1 MU/l, was bei einer spez. Aktivität von ca. 100 U/mg einer Expressionsleistung von > 10 g/l an aktiver, löslicher Hydantoinase entspricht.

### Beispiel 3

### 3.1 Aufschluß

1,5 kg Biofeuchtmasse werden mit 6 Liter kaltem 50 mmol/l TRIS/HCl - Puffer pH 8,5 suspendiert und mit einem Hochdruckhomogenisator der Firma APV Gaulin GmbH bei 1200 bar aufgeschlossen.
Die Suspension wird anschließend auf +4°C abgekühlt und hochtourig bei ca. 25 000 x g in einer Sorvallzentrifuge zentrifugiert.

### 3.2 Ammoniumsulfat-Fraktionierung

Der Rohextrakt wird mit festem Ammoniumsulfat bis zu einer Konzentration von 1,3 mol/l versetzt, der ausgefallene Niederschlag hochtourig abzentrifugiert verworfen. Der Überstand wird weiter mit Ammoniumsulfat bis zu einer Konzentration von 2,5 mol/l ausgefällt und der Niederschlag wiederum hochtourig abzentrifugiert.

### 3.3 Hitzeschritt

Der Niederschlag der Ammoniumsulfatfällung wird mit 50 mmol/l TRIS/HCl-Puffer pH 8,5 gelöst und auf eine Proteinkonzentration von 10 mg/ml eingestellt. Die Enzymlösung wird auf 56°C erwärmt und 30 Minuten bei dieser Temperatur gehalten; anschließend wird auf +4°C abgekühlt und der ausgefallene Niederschlag abzentrifugiert.

Die Ausbeute beträgt ca. 1 x 10⁶ Units mit einer spezifischen Aktivität von 35 U/mg Protein.

### 3.4 Vergleich mit dem Stand der Technik

Tabelle 1 zeigt einen Vergleich der Literaktivitäten für die bekannten D-Hydantoinasen und das erfindungsgemäße Enzym.

**Tabelle 1**

| D-Hydantoinase | Aktivität in kU/l |
|---|---|
| nach DE-A 30 31 151 (Wildtyp) | 0,10 |
| nach EP-B 0 219 034 | 0,56 |
| erfindungsgemäß | 1000 |

## Patentansprüche

1. DNA, welche für ein Protein mit D-Hydantoinasenaktivität codiert und ausgewählt ist aus der Gruppe
a) der in Sequenz SEQ ID NO 2 gezeigten DNA-Sequenz oder der dazu komplementären DNA-Sequenz,
b) DNA-Sequenzen, die aufgrund der Degeneration des genetischen Codes für ein Protein gemäß SEQ ID NO 1 codieren, welches auch von einer der in a) definierten Sequenzen codiert wird.

2. DNA gemäß Anspruch 1, welche die Sequenz SEQ ID NO 2 aufweist.

3. Verwendung einer DNA gemäß Anspruch 1 oder 2 zur Herstellung eines hitzestabilen Enzyms mit D-Hydantoinasenaktivität.

4. Prokaryontische oder eukaryontische Wirtszelle, die mit einer DNA-Sequenz nach den Ansprüchen 1 oder 2 so transformiert ist, daß die Wirtszelle das von dieser DNA-Sequenz codierte Protein exprimieren kann.

5. Wirtszelle nach Anspruch 4, **dadurch gekennzeichnet, daß** diese eine E.coli-Zelle oder eine Saccharomyces-Zelle ist.

6. DNA-Vektor, enthaltend eine Sequenz nach den Ansprüchen 1 oder 2.

7. Verfahren zur Herstellung einer rekombinanten D-Hydantoinase durch Transformation einer geeigneten Wirtszelle mit einer DNA nach den Ansprüchen 1 oder 2 in einem geeigneten Expressionssystem, Kultivierung der transformierten Wirtszellen und Isolierung der gebildeten D-Hydantoinase aus den Zellen oder dem Zellüberstand.

## Claims

1. DNA that codes for a protein with D-hydantoinase activity and is selected from the group
a) the DNA sequence shown in the sequence SEQ ID NO 2 or the complementary DNA sequence thereto,
b) DNA sequences which due to the degeneracy of the genetic code, code for a protein according to SEQ ID NO 1 which is also coded by one of the sequences defined in a).

2. DNA as claimed in claim 1 which has the sequence SEQ ID NO 2.

3. Use of a DNA as claimed in claim 1 or 2 to produce a heatstable enzyme with D-hydantoinase activity.

4. Prokaryotic or eukaryotic host cell that is transformed with a DNA sequence as claimed in claims 1 or 2 in such a way that the host cell expresses the protein coded by this DNA sequence.

5. Host cell as claimed in claim 4, wherein this is an E. coli cell or a Saccharomyces cell.

6. DNA vector containing a sequence as claimed in claims 1 or 2.

7. Process for the production of a recombinant D-hydantoinase by transforming a suitable host cell with a DNA as claimed in claims 1 or 2 in a suitable expression system, culturing the transformed host cells and isolating the D-hydantoinase that forms from the cells or the cell supernatant.

## Revendications

1. ADN qui code pour une protéine à activité de D-hydantoïnase et qui est choisi parmi le groupe comprenant
a) la séquence d'ADN indiquée dans la séquence SEQ ID N° 2 ou la séquence d'ADN complémentaire à la première citée,
b) des séquences d'ADN qui, sur base de la dégénération du code génétique, codent pour une protéine selon SEQ ID N° 1 qui est également codée par une des séquences définies dans a).

2. ADN selon la revendication 1, qui présente la séquence SEQ ID N° 2.

3. Utilisation d'un ADN selon la revendication 1 ou 2 pour la préparation d'une enzyme stable à la chaleur possédant une activité de D-hydantoïnase.

4. Cellule hôte procaryote ou eucaryote qui a été transformée avec une séquence d'ADN selon la revendication 1 ou 2 de telle sorte que la cellule hôte peut exprimer la protéine codée par cette séquence d'ADN.

5. Cellule hôte selon la revendication 4, **caractérisée en ce qu'**il s'agit d'une cellule E.coli ou d'une cellule Saccharomyces.

6. Vecteur d'ADN contenant une séquence d'ADN selon les revendications 1 ou 2.

7. Procédé pour la préparation d'une D-hydantoïnase recombinante par transformation d'une cellule hôte appropriée avec un ADN selon les revendications 1 ou 2 dans un système d'expression approprié, culture des cellules hôtes transformées et isolation de la D-hydantoïnase obtenue par rapport aux cellules ou au produit surnageant.


### SEQUENZ PROTOKOLL

(1) ALGEMEINE INFORMATION:
   (i) ANMELDER:
      (A) NAME: BOEHRINGER MANNHEIM GMBH
      (B) STRASSE: Sandhofer Str. 116
      (C) ORT: Mannheim
      (D) BUNDESLAND: BW
      (E) LAND: Germany
      (F) POSTLEITZAHL: D-68305
      (G) TELEPHON: 0621/759-3197
      (H) TELEFAX: 0621/759-4457
   (ii) ANMELDETITEL: Rekombinante D-Hydantoinase, Verfahren zur Herstellung und Verwendung
   (iii) ANZAHL DER SEQUENZEN: 4
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 460 Aminosäuren
      (B) ART: Aminosäure
      (C) STRANGFORM: Einzel
      (D) TOPOLOGIE: linear
   (xi) SEQUENZBESCHREIBUNG: SEQ ID NO: 1:
   Met Thr Lys Ile Ile Lys Asn Gly Thr Ile Val Thr Ala Thr Asp Thr
   1               5                   10                  15
   Tyr Glu Ala Asp Leu Leu Ile Lys Asp Gly Lys Ile Ala Met Ile Gly
               20                  25                  30
   Gln His Leu Glu Glu Lys Gly Ala Glu Val Ile Asp Ala Lys Gly Cys
           35                  40                  45
   Tyr Val Phe Pro Gly Gly Ile Asp Ser His Thr His Leu Asp Met Pro
       50                  55                  60
   Phe Gly Gly Thr Val Thr Lys Asp Asp Phe Glu Ser Gly Thr Ile Ala
   65 70 75 80
   Ala Ala Phe Gly Gly Thr Thr Thr Ile Ile Asp Phe Cys Leu Thr Asn
   85 90 95
   Lys Gly Glu Pro Leu Lys Lys Ala Ile Glu Thr Trp His Asn Lys Ala
   100 105 110
   Lys Gly Lys Ala Val Ile Asp Tyr Gly Phe His Leu Met Ile Ser Glu
   115 120 125
   Ile Thr Asp Asp Val Leu Glu Glu Leu Pro Lys Val Ile Ala Glu Glu
   130 135 140
   Gly Ile Thr Ser Phe Lys Val Phe Met Ala Tyr Lys Asn Val Phe Gln
   145 150 155 160
   Ala Asp Asp Gly Thr Leu Tyr Arg Thr Leu Val Ala Ala Lys Glu Leu
   165 170 175
   Gly Ala Leu Val Met Val His Ala Glu Asn Gly Asp Val Ile Asp Tyr
   180 185 190
   Leu Thr Lys Lys Ala Leu Ala Glu Gly Asn Thr Glu Pro Ile Tyr His
   195 200 205
   Ala Leu Thr Arg Pro Pro Glu Val Glu Gly Glu Ala Thr Gly Arg Ala
   210 215 220
   Cys Gln Leu Thr Glu Leu Ala Gly Ser Gln Leu Tyr Val Val His Val
   225 230 235 240
   Thr Cys Ala Gln Ala Val Glu Lys Ile Ala Gln Ala Arg Asn Lys Gly
   245 250 255
   Leu Asp Val Trp Gly Glu Thr Cys Pro Gln Tyr Leu Val Leu Asp Gln
   260 265 270
   Ser Tyr Leu Glu Lys Pro Asp Phe Glu Gly Ala Lys Tyr Val Trp Ser
   275 280 285
   Pro Pro Leu Arg Glu Lys Trp His Gln Glu Val Leu Trp Asn Ala Leu
   290 295 300
   Lys Asn Gly Gln Leu Gln Thr Leu Gly Ser Asp Gln Cys Ser Phe Asp
   305 310 315 320
   Phe Lys Gly Gln Lys Glu Leu Gly Arg Gly Asp Phe Thr Lys Ile Pro
   325 330 335
   Asn Gly Gly Pro Met Val Glu Asp Arg Val Ser Ile Leu Phe Ser Glu
               340                 345                 350
   Gly Val Lys Lys Gly Arg Ile Thr Leu Asn Gln Phe Val Asp Ile Met
           355                 360                 365
   Ser Thr Arg Ile Ala Lys Leu Phe Gly Leu Phe Pro Arg Lys Gly Thr
       370                 375                 380
   Ile Ala Val Gly Ser Asp Ala Asp Leu Val Ile Phe Asp Pro Asp Ile
   385                 390                 395                 400
   Glu Arg Val Ile Ser Ala Glu Thr His His Met Ala Val Asp Tyr Asn
                   405                 410                 415
   Ala Phe Glu Gly Met Lys Val Thr Gly Glu Pro Val Ser Val Leu Cys
               420                 425                 430
   Arg Gly Glu Phe Val Val Arg Asp Lys Gln Phe Val Gly Lys Pro Gly
           435                 440                 445
   Tyr Gly Gln Tyr Leu Lys Ala Gly Cys Phe Gly Gly
       450                 455                 460
(2) INFORMATION ZU SEQ ID NO: 2:
   (i) SEQUENZ CHARAKTERISTIKA:
      (A) LÄNGE: 1383 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 2:
   ATGACAAAAA TAATAAAAAA TGGAACGATT GTTACCGCAA CCGATACGTA TGAAGCGGAC   60
   TTGCTCATTA AAGACGGAAA AATTGCCATG ATAGGCCAAC ATTTAGAAGA AAAAGGCGCT  120
   GAAGTGATTG ATGCCAAAGG CTGTTACGTA TTTCCAGGCG GTATTGATTC GCACACGCAT  180
   TTAGATATGC CGTTTGGCGG CACGGTGACA AAGGATGATT TCGAATCTGG AACGATTGCG  240
   GCGGCATTTG GCGGAACAAC GACCATCATC GACTTTTGTT TAACGAATAA AGGGGAGCCA  300
   TTAAAAAAAG CGATTGAAAC TTGGCACAAC AAAGCGAAGG GAAAAGCGGT TATTGATTAT  360
   GGCTTCCATT TAATGATTAG CGAAATTACG GATGACGTAT TAGAAGAGCT GCCAAAAGTC  420
   ATTGCCGAAG AAGGGATAAC ATCCTTTAAA GTGTTTATGG CGTATAAAAA CGTATTTCAG  480
   GCAGATGATG GAACGTTATA CCGCACGCTA GTGGCTGCCA AAGAACTTGG CGCGCTTGTC  540
   ATGGTTCATG CGGAAAATGG GGATGTGATT GATTACTTAA CGAAAAAAGC GCTTGCGGAA  600
   GGGAATACGG AGCCGATTTA CCATGCTTTA ACGCGGCCTC CAGAAGTAGA AGGAGAAGCG  660
   ACCGGGCGCG CCTGTCAATT GACAGAGCTT GCCGGTTCAC AACTTTACGT TGTTCACGTG  720
   ACATGTGCGC AAGCGGTGGA AAAAATTGCA CAAGCGCGCA ATAAAGGGTT GGATGTGTGG  780
   GGAGAAACGT GTCCGCAATA TCTTGTTCTC GACCAATCGT ATTTAGAAAA GCCTGATTTT  840
   GAAGGCGCGA AATATGTTTG GTCCCCTCCG CTTCGTGAAA AATGGCATCA AGAAGTATTG  900
   TGGAATGCGC TGAAAAACGG CCAGCTGCAA ACGCTTGGAT CGGACCAATG TTCATTTGAC  960
   TTTAAAGGCC AAAAAGAACT TGGCAGAGGA GATTTTACTA AAATTCCAAA CGGCGGGCCG 1020
   ATGGTCGAGG ATCGGGTCAG CATTCTTTTC AGTGAAGGGG TTAAAAAAGG AAGAATCACG 1080
   TTAAATCAAT TTGTCGATAT TATGTCGACA AGAATTGCCA AATTGTTCGG GTTATTCCCG 1140
   AGAAAAGGAA CGATCGCGGT AGGTTCAGAC GCAGACTTAG TCATTTTTGA CCCGGATATC 1200
   GAACGGGTGA TTTCGGCGGA AACACACCAT ATGGCCGTCG ACTATAATGC ATTTGAAGGA 1260
   ATGAAAGTAA CGGGTGAACC GGTATCGGTT CTGTGCAGAG GCGAATTTGT TGTCCGTGAT 1320
   AAACAATTTG TCGGAAAACC AGGGTACGGC CAATATTTAA AAGCTGGCTG TTTTGGCGGA 1380
   TGA                                                               1383
(2) INFORMATION ZU SEQ ID NO: 3:
   (i) SEQUENZ CHARAKTERISTIKA:
      (A) LÄNGE: 31 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 3:
   GGAATTCTAT GACAAAAATA ATAAAAAATG G                                31
(2) INFORMATION ZU SEQ ID NO: 4:
   (i) SEQUENZ CHARAKTERISTIKA:
      (A) LÄNGE: 33 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 4:
   GCGGATCCAA GCTTTTAAAT ATTGGCCGTA CCC 33